(19) Europäisches Patentamt
European Patent Office
Office européen des brevets

(11) **EP 4 180 532 A1**

(12) **EUROPEAN PATENT APPLICATION**

(43) Date of publication:
**17.05.2023 Bulletin 2023/20**

(21) Application number: **22203021.5**

(22) Date of filing: **09.11.2016**

(51) International Patent Classification (IPC):
***C12Q 1/68*** *(2018.01)*  ***C12Q 1/6869*** *(2018.01)*

(52) Cooperative Patent Classification (CPC):
(C-Sets available)
**C12Q 1/6869**  (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **09.11.2015 US 201562252965 P**

(62) Document number(s) of the earlier application(s) in
accordance with Art. 76 EPC:
**16802196.2 / 3 374 521**

(71) Applicant: **Roche Molecular Systems, Inc.
Pleasanton, CA 94588 (US)**

(72) Inventor: **MANN, Tobias
San Diego, CA 92122 (US)**

(74) Representative: **Mewburn Ellis LLP
Aurora Building
Counterslip
Bristol BS1 6BX (GB)**

Remarks:
•This application was filed on 21.10.2022 as a
divisional application to the application mentioned
under INID code 62.
•Claims filed after the date of filing of the application
/ after the date of receipt of the divisional application
(Rule 68(4) EPC).

(54) **METHODS FOR DETERMINING THE ORIGIN OF DNA MOLECULES**

(57) The invention provides methods and nucleic acid molecules for determining the presence of DNA molecules from an origin of interest in a subject.

EP 4 180 532 A1

(52) Cooperative Patent Classification (CPC): (Cont.)

C-Sets
**C12Q 1/6869, C12Q 2535/122, C12Q 2537/165**

**Description**

**Cross Reference to Related Application**

This application claims the benefit of U.S. Provisional Application No. 62/252,965, filed on November 9, 2015, which is hereby incorporated herein by reference in its entirety.

**Field of the Invention**

This invention relates to systems and methods for determining, *inter alia,* the presence of DNA molecules from an origin of interest in a population of DNA molecules present in a cell-free bodily fluid sample from a subject.

**Background of the Invention**

Detection of the presence of DNA molecules from an origin of interest in a population of DNA molecules present in a cell-free bodily fluid sample from a subject can provide important diagnostic information to a physician. For example, noninvasive prenatal testing often relies on an estimate of the fetal DNA fraction present in a sample, rather than an empirically derived measurement of the fetal fraction. Having a definitive measurement of fetal fraction would allow physicians to make more accurate diagnoses of prenatal diseases and conditions. Current methods for determining fetal fraction are time-consuming or expensive, making them challenging to implement in noninvasive prenatal testing. Therefore, there is a need for developing cost-effective and efficient tests that have high sensitivities and specificities.

**Summary of the Invention**

Some embodiments of the invention are:

1. A method for determining the presence of DNA molecules from an origin of interest in a population of DNA molecules present in a cell-free bodily fluid sample from a subject, said method comprising:

a) obtaining a DNA sample isolated from a cell-free bodily fluid sample from the subject;
b) determining a plurality of protein binding site sequences and their 5' and 3' flanking region sequences for each of one or more proteins, wherein at least one of the one or more proteins differentially binds to DNA molecules of differing origin;
c) aligning at least a plurality of the determined protein binding site sequences for each of the one or more proteins;
d) counting the number of sequencing reads starting at each nucleotide position within each 5' and 3' flanking region sequence of the aligned protein binding site sequences;
e) generating a coverage map based on the number of counts of step d);
f) filtering the coverage map to identify at least one periodic component within the coverage map;
g) obtaining a metric that is representative of a strength of the at least one periodic component within the coverage map;

wherein the computed metric is indicative of the presence of DNA molecules from the origin of interest.
2. The method of embodiment 1, wherein the bodily fluid sample is a blood sample.
3. The method of embodiment 2, wherein the blood sample is from a pregnant woman.
4. The method of any one of embodiments 1-3, wherein the DNA molecules of differing origin are DNA molecules of maternal origin and DNA molecules of fetal origin.
5. The method of embodiment 4, wherein the computed metric is indicative of fetal DNA fraction.
6. The method of embodiment 1, wherein the DNA molecules of differing origin are DNA molecules of diseased cells and DNA molecules of non-diseased cells.
7. The method of embodiment 1, wherein the DNA molecules of differing origin are DNA molecules of a first tissue origin and DNA molecules of a second tissue origin.
8. The method of embodiment 1, wherein the DNA molecules of differing origin are DNA molecules of a first tissue origin and DNA molecules of leukocyte origin.
9. The method of any one of embodiments 1-8, wherein the determining is performed by sequencing.
10. The method of embodiment 9, wherein the sequencing is massively parallel sequencing.
11. The method of embodiment 9, wherein the sequencing is targeted sequencing.
12. The method of any one of embodiments 1-11, wherein the proteins are transcription factors and the protein binding site sequences are transcription factor binding site sequences.

13. The method of any one of embodiments 1-11, wherein the proteins are nucleases and the protein binding site sequences are nuclease binding sequences.

14. The method of any one of embodiments 1-13, wherein the aligning is an alignment against a genomic reference sequence.

15. The method of any one of embodiments 1-14, wherein the plurality of protein binding site sequences comprises at least 500, at least 1,000, at least 1,500, at least 2,000, at least 3,000, at least 4,000, at least 5,000, at least 10,000, at least 20,000, at least 30,000, at least 40,000, at least 50,000, at least 60,000, at least 70,000, at least 80,000, at least 90,000, at least 100,000, at least 110,000, at least 120,000, at least 130,000, at least 140,000, at least 150,000, at least 160,000, at least 170,000, at least 180,000, at least 190,000, at least 200,000, at least 210,000, at least 220,000, at least 230,000, at least 240,000, at least 250,000, at least 260,000, at least 270,000, at least 280,000, at least 290,000, at least 300,000, at least 310,000, at least 320,000, at least 330,000, at least 340,000, at least 350,000, at least 360,000, at least 370,000, at least 380,000, at least 390,000, at least 400,000, at least 410,000, at least 420,000, at least 430,000, at least 440,000, at least 450,000, at least 460,000, at least 470,000, at least 480,000, at least 490,000, or at least 500,000 protein binding site sequences.

16. The method of any one of embodiments 1-15, wherein the one or more proteins is two proteins.

17. The method of any one of embodiments 1-15, wherein the one or more proteins is three proteins.

18. The method of any one of embodiments 1-15, wherein the one or more proteins is four proteins.

19. The method of any one of embodiments 1-15, wherein the one or more proteins is five proteins.

20. The method of any one of embodiments 1-15, wherein the one or more proteins is 6, 7, 8, 9, 10, 11, 12, 13, 14, 15, 16, 17, 18, 19, 20, or 21 or more proteins.

21. The method of any one of embodiments 1-20, wherein the 5' and 3' flanking region sequences are at least 200, at least 300, at least 400, at least 500, at least 600, at least 700, at least 800, at least 900, at least 1,000, at least 1,100, at least 1,200, at least 1,300, at least 1,400, at least 1,500, or at least 2,000 base pairs.

22. The method of any one of embodiments 1-21, wherein the filtering of step f) comprises computing a spectral frequency transform of the coverage map and identifying a power of the spectral frequency transform within a frequency band.

23. The method of embodiment 22, wherein the frequency band includes frequencies corresponding to spacings of 130 to 250 base pairs.

24. The method of any one of embodiments 22 or 23, wherein the metric is a ratio between the power of the spectral frequency transform within a frequency band and an overall power of the spectral frequency transform.

25. The method of embodiment 24, wherein the power of the spectral frequency transform is computed by integrating the spectral frequency transform within the frequency band, and the overall power of the spectral frequency transform is computed by integrating the spectral frequency transform over all frequencies.

26. The method of any one of embodiments 1-25, wherein the at least one periodic component is indicative of aligned positions across nucleosomes, such that a local maximum in the at least one periodic component is indicative of an absence of nucleosomes at the corresponding nucleotide position, and a local minimum in the at least one periodic component is indicative of a presence of nucleosomes at the corresponding nucleotide position.

27. The method of any one of embodiments 1-26, wherein the metric is a signal-to-noise ratio that is computed from the filtered coverage map.

28. The method of any one of embodiments 1-27, further comprising determining a proportion of DNA molecules from two or more origins of interest.

29. The method of embodiment 28, wherein the two or more origins of interest are tissues.

**Brief Description of the Drawings**

**Figure 1** depicts a transcription factor (TF) and nucleosomes positioned on DNA.

**Figures 2A and 2B** depict DNA protection and a coverage map.

**Figure 3** depicts a coverage map for the 5' and 3' flanking regions around the CTCF transcription factor (TF) binding site.

**Figure 4** depicts a control coverage map. The control coverage map corresponds to nucleotide positions that are right-shifted by 2000 base pairs compared to the CTCF coverage map shown in Figure 3.

**Figures 5A-5D** depict the coverage maps for CTCF, E2F1, GTF2F1, and EBF1, respectively.

**Figures 6A-6E** depict five different coverage maps for CTCF, wherein each of the five coverage maps corresponds to a different number of binding sites.

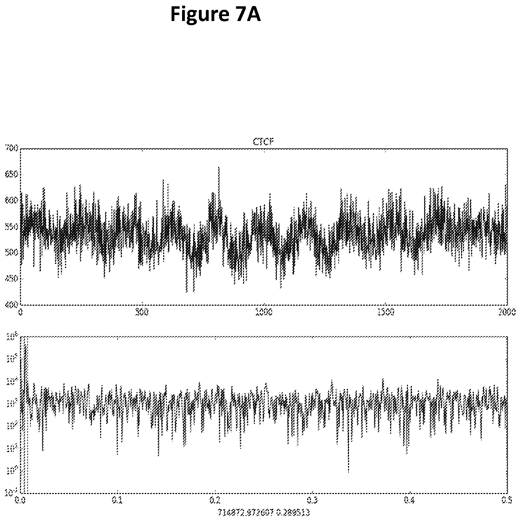

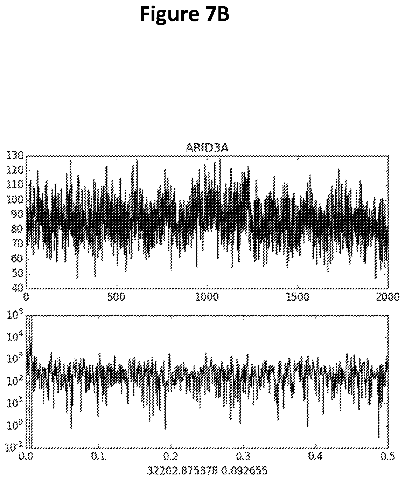

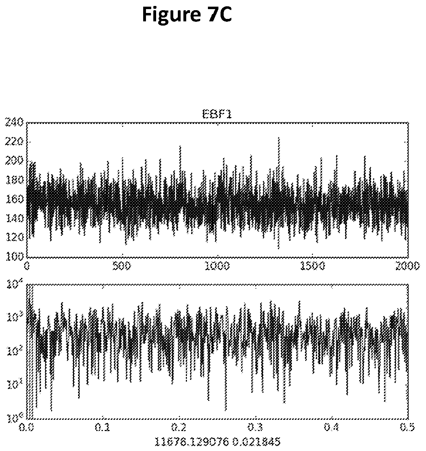

**Figures 7A-7C** depict panels corresponding to transcription factors CTCF, ARID3A, and EBF1, respectively. In each figure, the top panel depicts a coverage map, and the bottom panel depicts a corresponding frequency transform.

**Figures 8A-8C** depict charts showing the relative preference of SPI1, FOXM1, and MAZ, respectively, for binding to DNA molecules of fetal or maternal origin.

**Figure 9** shows panels depicting the correlation between the predicted fraction and the y-fraction.

## Detailed Description of the Invention

This invention provides a system and method for determining the presence of DNA molecules from an origin of interest in a population of DNA molecules present in a cell-free bodily fluid sample from a subject.

In order that these inventions and their embodiments herein described may be fully understood, the following detailed description is set forth.

Unless otherwise defined herein, scientific and technical terms used in this application shall have the meanings that are commonly understood by those of ordinary skill in the art to which this invention belongs. Generally, nomenclature used in connection with, and techniques of, cell and tissue culture, molecular biology, cell biology, cancer biology, neurobiology, neurochemistry, virology, immunology, microbiology, genetics, protein and nucleic acid chemistry, chemistry, and pharmacology described herein, are those well known and commonly used in the art. Each embodiment of the inventions described herein may be taken alone or in combination with one or more other embodiments of the inventions.

The methods and techniques of the present inventions and their embodiments are generally performed, unless otherwise indicated, according to methods of molecular biology, cell biology, biochemistry, microarray and sequencing technology well known in the art and as described in various general and more specific references that are cited and discussed throughout this specification. See, *e.g.* Motulsky, "Intuitive Biostatistics", Oxford University Press, Inc. (1995); Lodish et al., "Molecular Cell Biology, 4th ed.", W. H. Freeman & Co., New York (2000); Griffiths et al., "Introduction to Genetic Analysis, 7th ed.", W. H. Freeman & Co., N.Y. (1999); Gilbert et al., "Developmental Biology, 6th ed.", Sinauer Associates, Inc., Sunderland, MA (2000).

Chemistry terms used herein are used according to conventional usage in the art, as exemplified by "The McGraw-Hill Dictionary of Chemical Terms", Parker S., Ed., McGraw-Hill, San Francisco, C.A. (1985).

All of the above, and any other publications, patents and published patent applications referred to in this application are specifically incorporated by reference herein. In case of conflict, the present specification, including its specific definitions, will control.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to". "Including" and "including but not limited to" are used interchangeably.

It will be understood by one of ordinary skill in the art that the compositions and methods described herein may be adapted and modified as is appropriate for the application being addressed and that the compositions and methods described herein may be employed in other suitable applications, and that such other additions and modifications will not depart from the scope hereof.

These inventions and their embodiments will be better understood from the Experimental Details which follow. However, one skilled in the art will readily appreciate that the specific methods and results discussed are merely illustrative of the inventions and their embodiments which follow thereafter.

Methods for determining the presence of DNA molecules from an origin of interest

Current approaches for estimating fetal fraction are either based on single nucleotide polymorphisms (SNPs) or on DNA fragments. In SNP-based techniques, fetal fraction is determined by analyzing variants present in circulating cell-free fetal (cff) DNA that are heterozygous in the fetus and homozygous in the maternal genome. However, this approach requires very high coverage at variant sites. By contrast, in fragment-based approaches, fetal fraction is estimated by ascertaining the distribution of lengths of DNA fragments in a sample. However, this approach requires long read or paired end sequencing, or another method for measuring the distribution of fragment lengths, and is less economical than single end sequencing.

The embodiments of these inventions provide methods of determining fetal fraction using protein binding sites present in DNA. These methods, in addition to being useful for determining fetal fraction, also can be used more generally to determine the presence of DNA molecules from an origin of interest in a population of DNA molecules present in a cell-free bodily fluid sample from a subject. This determination is possible because of the ordering of DNA around certain sequences. The ordering is different in DNA molecules from different origins (e.g., different tissues), and therefore, detection of the ordering around certain sequences provides information on the origin of the DNA. For example, nucleosomes may become ordered around a variety of types of sequences, but typically become ordered during chromatin remodeling as the DNA is unwound. For example, when transcription factors bind to DNA, the surrounding nucleosomes become more ordered around the transcription factor binding site. Similarly, nucleosomes become more ordered near nuclease binding sites upon nuclease binding. Exemplary nuclease binding sites are DNAse-I hypersensitivity sites and MNAse hypersensitivity sites.

Some embodiments provide a method for determining the presence of DNA molecules from an origin of interest in a population of DNA molecules present in a cell-free bodily fluid sample from a subject, the method comprising:

a) obtaining a DNA sample isolated from a cell-free bodily fluid sample from the subject;

b) determining a plurality of protein binding site sequences and their 5' and 3' flanking region sequences for each of one or more proteins, wherein at least one of the one or more proteins differentially binds to DNA molecules of differing origin;

c) aligning at least a plurality of the determined protein binding site sequences for each of the one or more proteins;

d) counting the number of sequencing reads starting at each nucleotide position within each 5' and 3' flanking region sequence of the aligned protein binding site sequences;

e) generating a coverage map based on the number of counts of step d);

f) filtering the coverage map to identify at least one periodic component within the coverage map;

g) obtaining a metric that is representative of a strength of the at least one periodic component within the coverage map;

wherein the computed metric is indicative of the presence of DNA molecules from the origin of interest.

Obtaining a sample and sample preparation

Certain aspects or embodiments encompass obtaining a cell-free bodily fluid sample (e.g., a cell-free blood sample) containing DNA molecules from a subject. The term "sample", as used herein, refers to a sample typically derived from a biological fluid, cell, tissue, organ, or organism. It comprises a nucleic acid or a mixture of nucleic acids, comprising at least one nucleic acid sequence. Samples include, but are not limited to blood, whole blood, a blood fraction, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, chorionic villus sample, fluid from a preimplantation embryo, maternal urine, maternal saliva, placental sample, fetal blood, lavage and cervical vaginal fluid, interstitial fluid, ocular fluid, sputum/oral fluid, amniotic fluid, or fine needle biopsy samples (e.g., surgical biopsy, fine needle biopsy, etc.) peritoneal fluid, and the like. Exemplary blood samples include, but are not limited to, a blood sample such as a whole blood sample, a serum sample, or a plasma sample. A cell-free sample may be derived from any of the above types of samples. For example, a cell-free blood sample may be derived from a whole blood sample by removing cells from the whole blood sample. Cell-free blood samples include, but are not limited to, plasma and serum samples. In alternative embodiments, the sample may be a cell-free sample that is not a blood sample. Moreover, in certain aspects or embodiments, obtaining the DNA molecule-containing sample may include, for example, extracting or purifying DNA from the cell-free bodily fluid sample, or enriching the sample for DNA. In some embodiments, only specific sites may be of interest in the cell-free sample. In these embodiments, hybridization-based capture process can be designed to the sequences of interest, and the DNA to be sequenced can be enriched for sites of interest by first hybridizing the sample to the capture probes, and then recovering the hybridized material for sequencing,

The terms "subject" and "patient", as used herein, refer to any animal, such as a dog, a cat, a bird, livestock, and particularly a mammal, and preferably a human.

Although a sample is often taken from a human subject (e.g., patient), the sample can be taken from any mammal, including, but not limited to dogs, cats, horses, goats, sheep, cattle, pigs, etc. The sample may be used directly as obtained from the biological source or following a pretreatment to modify the character of the sample. For example, such pretreatment may include preparing plasma from blood, diluting viscous fluids and so forth. Methods of pretreatment may also involve, but are not limited to, filtration, precipitation, dilution, distillation, mixing, centrifugation, freezing, lyophilization, concentration, amplification, nucleic acid fragmentation, inactivation of interfering components, the addition of reagents, lysing, etc. Even when such methods of pretreatment are employed with respect to the sample, the nucleic acid(s) or DNA molecules of interest remain in the test sample, preferably at a concentration proportional to that in an untreated test sample (e.g., namely, a sample that is not subjected to any such pretreatment method(s)). Depending on the type of sample used, additional processing and/or purification steps may be performed to obtain nucleic acid fragments of a desired purity or size, using processing methods, including but not limited to, sonication, nebulization, gel purification, PCR purification systems, nuclease cleavage, size-specific capture or exclusion, targeted capture or a combination of these methods.

In some embodiments, the blood sample is from a pregnant woman. In other embodiments, the DNA molecules from an origin of interest are DNA molecules of maternal origin and DNA molecules of fetal origin. Some embodiments provide a method of determining fetal fraction based on the determination that one or more DNA molecules are of maternal origin and one or more molecules are of fetal origin. The fetal fraction may be determined based on a metric (e.g., a computed metric), as described in more detail below. In some embodiments, the DNA molecules from an origin of interest are DNA molecules of diseased cells and DNA molecules of non-diseased cells. In some embodiments, the DNA molecules from an origin of interest are DNA molecules of a first tissue and DNA molecules of a second tissue. In some embodiments, the DNA molecules from an origin of interest are DNA molecules of a first tissue origin and DNA molecules of leukocyte origin. Certain embodiments provide a method for detecting the presence of a cancer, e.g., liver cancer and/or lymphoma, that sheds cells or nucleic acids (e.g., DNA) into the blood. For example, a high proportion of DNA from the liver in the blood may indicate the presence of liver cancer. Likewise, certain embodiments provide a method for detecting bladder or kidney cancer, for example, by detecting DNA molecules from the bladder or kidneys in urine.

Moreover, a subject receiving a transplant (e.g., an organ transplant) may have increased levels of DNA molecules from the transplant in the blood, especially if the transplant is being rejected by the body. Thus, certain embodiments provide a method for detecting transplant rejection. Certain embodiments also provide a method for monitoring surgical recovery, organ failure, and/or tissue necrosis. Further embodiments also provide a method for diagnosing heart disease, for example, by detecting DNA molecules from the heart in the blood.

DNA organization

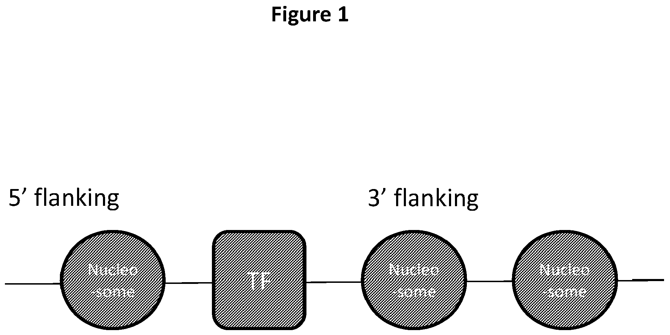

DNA is organized in certain regions of the genome (e.g., the organization of chromatin around transcription factor binding sites). This organization around specific sites differs in DNA obtained from different origins (e.g., DNA from different tissues will have different patterns of organization). Thus, DNA organization around specific sites can be used to determine the origin of the DNA. Moreover, because DNA organization can be a function of protein binding to DNA, differential protein binding between DNA molecules from differing origins of interest can be used to determine the origin of those molecules. As used herein, the terms "nucleic acid," "nucleic acid molecules," and "DNA molecules" encompass DNA, e.g., genomic DNA. In some embodiments, the DNA organization occurs around protein binding sites. Thus, the protein binding site will have 5' and 3' flanking regions with varying degrees of organization. For example, the 5 and 3' flanking regions may be more organized closer to the protein binding site and less organized further from the protein binding site. As used herein, a "protein binding site" is a DNA site to which a protein binds. Exemplary proteins useful in these embodiments include, but are not limited to, transcription factors and nucleases. When the protein binding site is a transcription factor binding site, DNA organization may be due to nucleosome organization around the transcription factor binding site. See, for example, Figure 1, which depicts a transcription factor (TF) and nucleosomes positioned on DNA. However, the further away from the transcription factor binding site one gets, the less organized the DNA may be. Without wishing to be bound by theory, this may be because the nucleosomes have some amount of variability with respect to DNA positioning. As the transcription factor binding site opens, the nucleosome are no longer able to move as freely, and thus, become more organized. The further one goes, the more freedom the nucleosomes will have to move. Moreover, nucleosome positioning from one DNA molecule to the next will vary slightly. Transcription factor binding reduces this variability between DNA molecules. Exemplary transcription factors include CTCF and myc (also known as c-myc). For example, myc binding sites may be used as a protein binding site to distinguish between DNA molecules originating from a cancer cell and DNA molecules originating from a non-cancer cell.

In some embodiments, the plurality of protein binding site sequences comprises at least 500, at least 1,000, at least 1,500, at least 2,000, at least 3,000, at least 4,000, at least 5,000, at least 10,000, at least 20,000, at least 30,000, at least 40,000, at least 50,000, at least 60,000, at least 70,000, at least 80,000, at least 90,000, at least 100,000, at least 110,000, at least 120,000, at least 130,000, at least 140,000, at least 150,000, at least 160,000, at least 170,000, at least 180,000, at least 190,000, at least 200,000, at least 210,000, at least 220,000, at least 230,000, at least 240,000, at least 250,000, at least 260,000, at least 270,000, at least 280,000, at least 290,000, at least 300,000, at least 310,000, at least 320,000, at least 330,000, at least 340,000, at least 350,000, at least 360,000, at least 370,000, at least 380,000, at least 390,000, at least 400,000, at least 410,000, at least 420,000, at least 430,000, at least 440,000, at least 450,000, at least 460,000, at least 470,000, at least 480,000, at least 490,000, or at least 500,000 protein binding site sequences.

In some embodiments, the methods described herein comprise determining the plurality of protein binding site sequences and their 5' and 3' flanking region sequences for each of one or more proteins, wherein the one or more proteins is two proteins. In some embodiments, the one or more proteins is three proteins. In some embodiments, the one or more proteins is four proteins. In some embodiments, the one or more proteins is five proteins. In some embodiments, the one or more proteins is 6, 7, 8, 9, 10, 11, 12, 13, 14, 15, 16, 17, 18, 19, 20, or 21 or more proteins.

In some embodiments, the determining of the plurality of protein binding site sequences and their 5' and 3' flanking region sequences for each of one or more proteins comprises sequencing. The term "sequencing", as used herein, is used in a broad sense and may refer to any technique known in the art that allows the order of at least some consecutive nucleotides in at least part of a nucleic acid to be identified, including without limitation at least part of an extension product or a vector insert. Sequencing also may refer to a technique that allows the detection of differences between nucleotide bases in a nucleic acid sequence. Exemplary sequencing techniques include targeted sequencing, single molecule real-time sequencing, electron microscopy-based sequencing, transistor-mediated sequencing, direct sequencing, random shotgun sequencing, Sanger dideoxy termination sequencing, targeted sequencing, exon sequencing, whole-genome sequencing, sequencing by hybridization (e.g., in an array such as a microarray), pyrosequencing, capillary electrophoresis, gel electrophoresis, duplex sequencing, cycle sequencing, single-base extension sequencing, solid-phase sequencing, high-throughput sequencing, massively parallel shotgun sequencing, emulsion PCR, co-amplification at lower denaturation temperature-PCR (COLD-PCR), multiplex PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, ion semiconductor sequencing, nanoball sequencing, nanopore sequencing, 454 sequencing, Solexa Genome Analyzer sequencing, miSeq (Illumina), HiSeq 2000 (Illumina), HiSeq 2500 (Illumina), Illumina Genome Analyzer (Illumina), Ion Torrent PGM™ (Life Technologies), MinION™ (Oxford Nanopore Technologies), real-time SMRT™ technology (Pacific Biosciences), the Probe-Anchor Ligation (cPAL™) (Complete Genomics/BGI), SOLiD® sequencing, MS-PET sequencing, mass spectrometry, and a combination thereof. In some embodiments, sequencing comprises detecting the sequencing product using an instrument, for example but not limited to an ABI PRISM® 377 DNA Sequencer, an ABI PRISM® 310, 3100, 3100-Avant, 3730, or 373OxI Genetic Analyzer, an ABI PRISM® 3700 DNA Analyzer, or an Applied Biosystems SOLiD™ System (all from Applied Biosystems), a Genome Sequencer 20 System (Roche Applied Science), or a mass spectrometer. In certain embodiments, sequencing comprises emulsion PCR. In certain embodiments, sequencing comprises a high throughput sequencing technique. In certain embodiments, sequencing comprises whole genome sequencing. In certain embodiments, sequencing comprises massively parallel sequencing (e.g., massively parallel shotgun sequencing). In alternative embodiments, sequencing comprises targeted sequencing.

The methods and apparatus described herein may alternatively employ enrichment-based technology instead of sequencing techniques.

In some embodiments, the 5' and 3' flanking region sequences are each at least 200, at least 300, at least 400, at least 500, at least 600, at least 700, at least 800, at least 900, at least 1,000, at least 1,100, at least 1,200, at least 1,300, at least 1,400, at least 1,500, or at least 2,000 base pairs. In certain embodiments, the 5' and 3' flanking region sequences are each 500-600 base pairs. In certain embodiments, the 5' and 3' flanking region sequences are each less than 1,000 base pairs. In certain embodiments, the 5' and 3' flanking region sequences are each 500-1,000 base pairs. In certain embodiments, the 5' and 3' flanking region sequences used in the methods of the invention are of the same length. In alternative embodiments, the 5' and 3' flanking region sequences used in the methods of the invention are of different lengths.

Alignment

In some embodiments, after determining a plurality of protein binding site sequences and their 5' and 3' flanking region sequences, at least a plurality of the determined protein binding site sequences for each of the one or more proteins are aligned (for example, using a genomic reference sequence). By aligning at least the determined protein binding site sequences, the skilled worker will appreciate that the 5' and 3' flanking sequences also may be aligned. Although a transcription factor may have many binding sites, the alignment of these sites is within the skill of the art. In some embodiments, after alignment of the protein binding site sequences and the 5' and 3' sequences, the protein binding site sequences are removed from the alignment, leaving the 5' and 3' sequences.

Counting sequencing reads and generating a coverage map

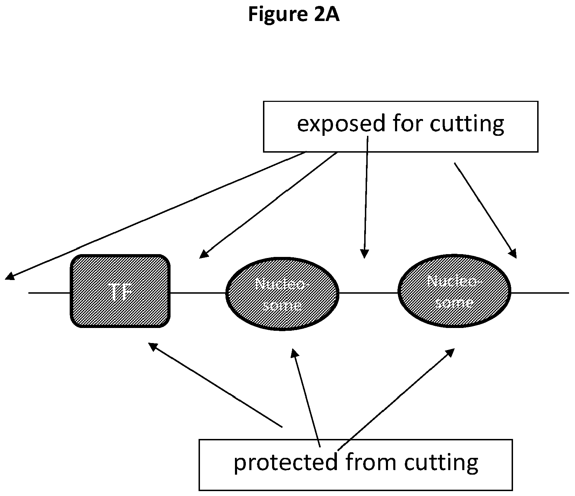

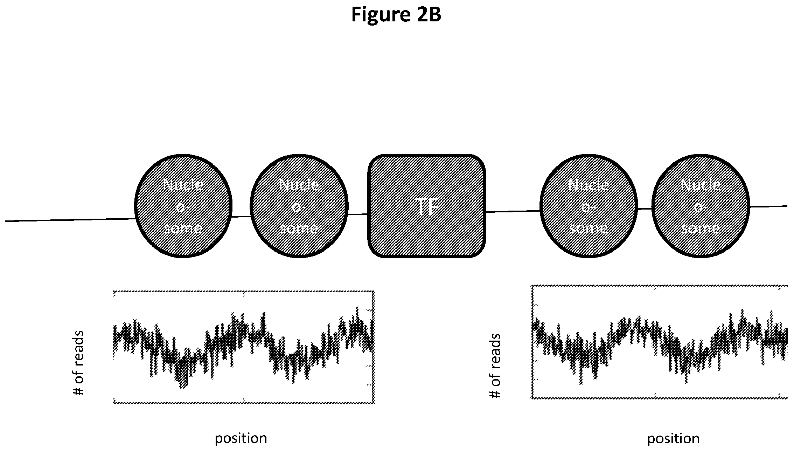

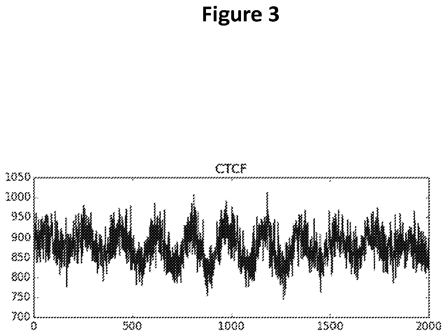

In some embodiments, after alignment, the number of sequencing reads starting at each nucleotide position within each 5' and 3' flanking region sequence of the aligned protein binding site sequences is counted. These counts are then used to generate a coverage map that indicates how many sequencing reads began at each nucleotide position in the DNA molecules. Counting the number of sequencing reads that starts at each nucleotide position helps indicate how the DNA is organized. For example, around a transcription factor binding site, nucleosomes will be bound to DNA in a regular pattern. Where the nucleosomes are bound, the DNA will be protected from degradation, which may occur naturally in the blood, for example, as part of apoptosis or necrosis, or as a result of the introduction of one or more DNA cleavage enzymes to a sample. Thus, the coverage map will show more reads beginning between nucleosomes (where DNA is unprotected) than in the regions where nucleosomes are bound (and where DNA is protected). See, for example, Figures 2A and 2B, which depict DNA protection and Figure 3, which depicts a coverage map for the 5' and 3' flanking regions around the CTCF transcription factor (TF) binding site.

A CTCF coverage map has a strong periodic component

The coverage map in Figure 3 depicts a strong periodic component observed in cell-free blood samples from pregnant women. The coverage map is generated by using the number of sequencing reads starting at each position starting 1000 base pairs before the CTCF binding site (positions 0-999 on the x-axis) (i.e., the 5' flanking region) and ending 1000 base pairs after the CTCF binding site (positions 1000-1999 on the x-axis) (i.e., the 3' flanking region). The CTCF binding site itself is omitted from the coverage map. The strong periodic component indicates that the transcription factor CTCF causes the nucleosomes across multiple CTCF sites to be well-positioned with or ordered in relation to one another. In other words, a local maximum in the coverage map indicates that a relatively large number of sequencing reads started at the corresponding nucleotide position, and a local minimum in the coverage map indicates that a relatively low number of sequencing reads started at the corresponding nucleotide position. A high number of sequencing read starts is indicative of an absence of nucleosomes at the corresponding nucleotide position, and a low number of sequencing read starts is indicative of the presence of nucleosomes at the corresponding nucleotide position. Accordingly, a strong periodic component having local maxima and minima is indicative that the positions of the nucleosomes are well-positioned across different sites for the same transcription factor.

The periodic component in the CTCF coverage map is determined to be strong, where 49% of the spectral power in the coverage map of Figure 3 is within the frequency band of interest. As is described in further detail below, the frequency band of interest may correspond to a nucleosomal frequency band, and may include frequencies corresponding to periods of 130 to 250 base pairs. As will be understood by one of ordinary skill in the art, this range is provided by way of example only, and other frequency bands corresponding to other spacings may be used without departing from the scope of the present disclosure.

The CTCF coverage map further indicates that the periodic component is stronger towards the center of the coverage map (from position 500 to 1500 on the horizontal axis), and weaker towards the far left hand side and far right hand side of the coverage map. The decreasing strength of periodicity as the position moves further from the binding site is indicative of poorer positioning of nucleosomes at further locations.

Testing for specificity of the periodicity to the protein binding sites

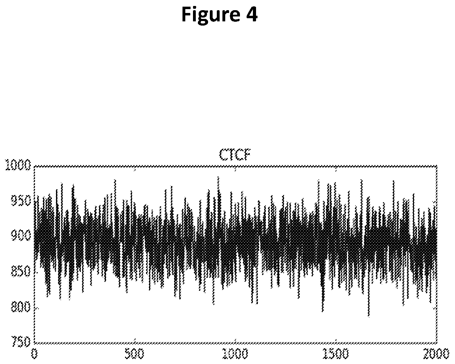

To determine whether a periodic signal that is observed in the CTCF coverage map (Figure 3) is specific to the protein binding sites corresponding to CTCF, a control coverage map (Figure 4) may be generated. The control coverage map is generated by counting the numbers of sequencing reads starting at nucleotide positions that are shifted to the right by 2000 base pairs. In other words, the control coverage map in Figure 4 corresponds to nucleotide positions that are right-shifted by 2000 base pairs compared to the CTCF coverage map shown in Figure 3. In the control coverage map, only 0.3% of the spectral power is within the frequency band of interest (e.g., corresponding to periods of 130-250 base pairs). The stark contrast between the CTCF coverage map in Figure 3 and the control coverage map in Figure 4 suggests that the binding of the CTCF transcription factor causes the nucleosomes near the CTCF binding sites to become well positioned across different CTCF binding sites, while the nucleosomes at other sites are more poorly positioned.

Coverage maps for different transcription factors exhibit different degrees of periodicity

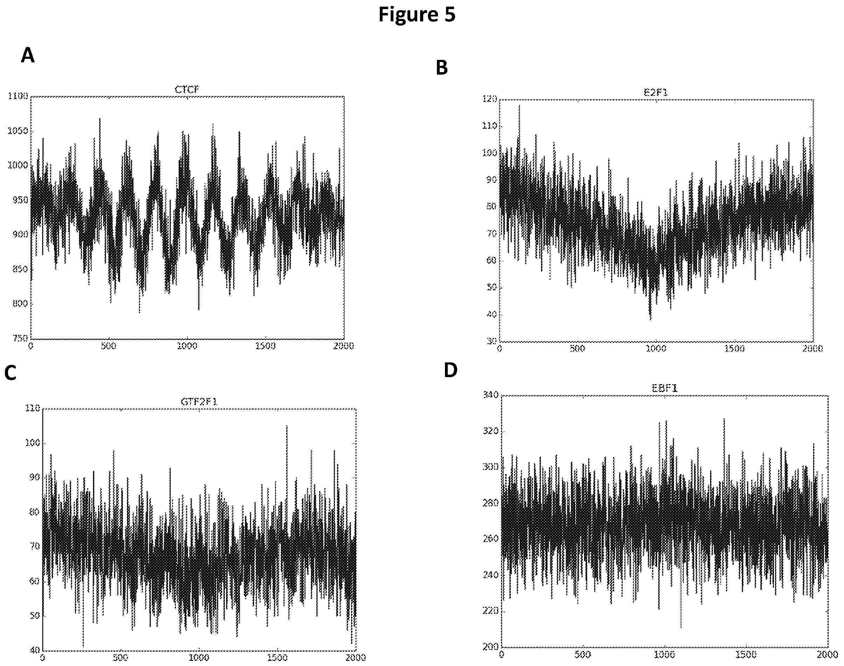

Coverage maps are generated for various transcription factors. The four plots in Figures 5A-5D depict the coverage maps for CTCF, E2F1, GTF2F1, and EBF1. The horizontal axis of each plot corresponds to a nucleotide position within the flanking regions, and the vertical axis of each plot corresponds to a number of total counts of sequencing reads that start at each nucleotide position. The horizontal axis varies from 0 to 2000, where the left half of the plot (e.g., from 0 to 999) corresponds to the 5' flanking region, and the right half of the plot (e.g., from 1000 to 1999) corresponds to the 3' flanking region.

As can be seen in the CTCF plot in Figure 5A, a strong periodic component is present in the coverage map. As was described above, the strong periodic component indicates that the transcription factor CTCF causes the nucleosomes across multiple CTCF binding sites to be well-positioned with one another.

By contrast, the three coverage maps for E2F1, GTF2F1 and EBF1 shown in Figures 5B, 5C, and 5D, respectively, show no obvious periodicity. The lack of periodicity in these three plots indicates that (1) the positions of the nucleosomes are not organized in the same manner that they were for CTCF, (2) there are not enough binding sites to sufficiently identify periodicity, or (3) both.

Determining how many binding sites are sufficient for identifying periodicity in the coverage map

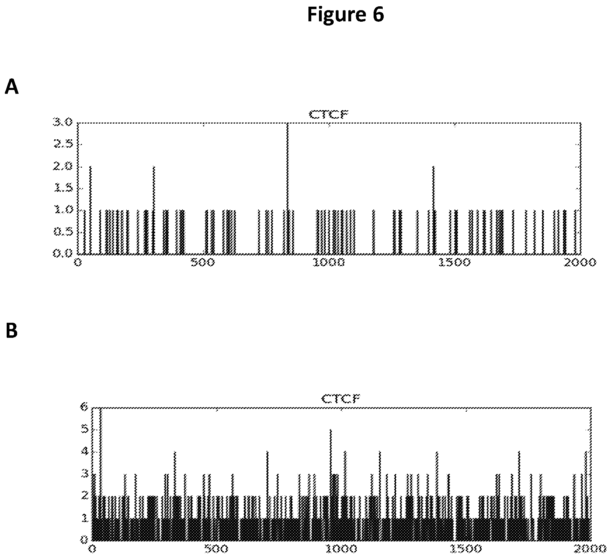

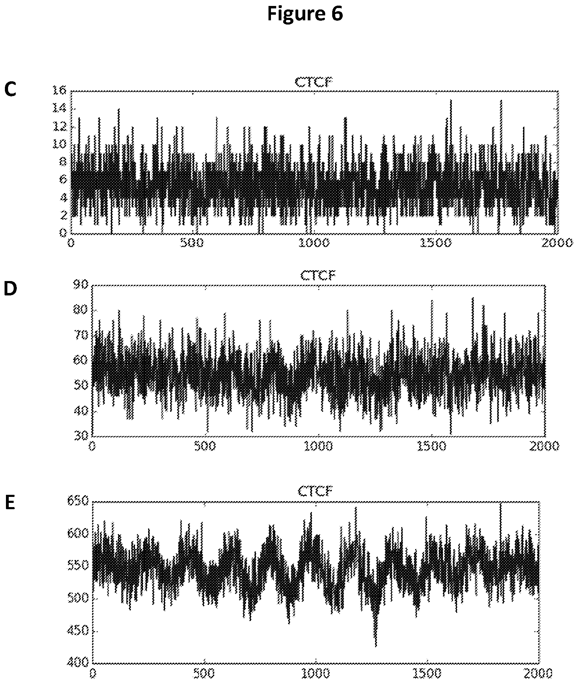

In some embodiments, a low number of binding sites may not produce a coverage map with a strong detectable periodic pattern. However, as the number of binding sites increases, the periodic pattern (if one exists, e.g., if the nucleosomes within the flanking regions are well positioned across different binding sites for the same transcription factor) should become more apparent. To determine a number of binding sites that would be sufficient to detect a periodic pattern, five different coverage maps are generated (shown in Figures 6A-6E), where each of the five coverage maps corresponds to a different number of binding sites. Rather than relying on a subjective analysis to determine whether periodicity exists in a coverage map or not, it is desirable to use a quantitative metric that is representative of a strength of periodicity in the coverage map. For each coverage map, the percentage spectral power within the frequency band of interest (e.g., corresponding to periods of 130-250 base pairs) is measured. Table 1 below indicates the number of sites and corresponding percentage spectral power for each of the five panels shown in Figures 6A-6E.

Table 1

| Panel | Number of sites | Percentage spectral power |
|-------|-----------------|---------------------------|
| A | 10 | 1.2% |
| B | 100 | 1.5% |
| C | 1,000 | 1.1% |
| D | 10,000 | 8% |
| E | 100,000 | 40% |

Statistical tests may be performed to determine whether a percentage spectral power is statistically different from a predetermined set of values, which may be around 1%. The results, as shown and as described above, indicate that a suitable threshold number of sites sufficient to identify a periodic pattern in the coverage map may be between 1,000 sites and 10,000 sites.

Measuring periodicity

In some embodiments, one or more periodic components of the coverage map are identified by filtering the coverage map, and a metric that is representative of a strength of the periodic component(s) is computed. In one example, filtering of the coverage map involves obtaining (e.g., by computing) a frequency transform of the coverage map and using the frequency transform to compute the metric. In particular, the metric may correspond to a signal-to-noise ratio, where the numerator of the ratio corresponds to the power of the frequency transform within a particular frequency band, and the denominator of the ratio corresponds to an overall power of the frequency transform. In other words, the ratio may correspond to the following expression:

$$\frac{\int_a^b |F(\omega)| d\omega}{\int |F(\omega)| d\omega}$$

where F(w) corresponds to the Fourier coefficient for frequency w, a corresponds to a first edge of the frequency band, and b correspond to a second edge of the frequency band. In an example, when the frequency band is a nucleosomal frequency band, a may be a frequency corresponding to a period of 250 base pairs, and b may be a frequency corresponding to a period of 130 base pairs. The numerator in the above expression is an integral of the frequency transform within a particular frequency band of interest, or the spectral power. In this way, the numerator is indicative of a periodicity within the coverage map, at periods corresponding to the band of frequencies. The denominator in the above expression is an integral of the frequency transform over all frequencies, and is representative of an overall power of the coverage map.

In some embodiments, the coverage map may be pre-processed before its frequency transform is computed. In an example, the coverage map may be processed to (1) compute the mean value of the coverage map and (2) subtract the mean value from the coverage map. By forcing the coverage map to be centered around zero, this ensures that the frequency transform has no DC component. Alternatively, if the coverage map is not centered around zero, then the DC component of the frequency transform may be removed before obtaining (e.g., by computing) the metric or determining the strength of the periodic component(s) of the coverage map.

Each of the three figures, 7A-7C, is for a particular transcription factor (CTCF, ARID3A, and EBF1). In each figure, the top panel depicts a coverage map, and the bottom panel depicts a corresponding frequency transform, where the amplitude is plotted on a log-scale, and the horizontal axis corresponds to frequency. Each bottom panel further includes two vertical lines indicative of the frequency band of interest (e.g., corresponding to "a", or 250 base pairs for the left red line and "b", or 130 base pairs for the right red line). Table 2 below indicates the percentage spectral power (e.g., the ratio as defined above) for each of the three transcription factors.

Table 2

| Transcription Factor | Percentage spectral power |
| --- | --- |
| CTCF | 29% |
| ARID3A | 9% |
| EBF1 | 2% |

While computing the frequency transform and measuring a power of the spectral transform within a particular frequency band is one way of measuring the periodicity in a coverage map, the periodicity may be measured in any of a number of other ways. For example, rather than performing the measurement in the frequency domain, an equivalent analysis may be performed in the space domain, by convolving the coverage map with a band pass filter in the space domain. A metric similar to the ratio that is described above may be computed by dividing the power of the waveform that results after the convolution by the power of the unconvolved coverage map. In another example, a strength of the periodicity of the coverage map may be computed by using match filters, gabor filters, wavelet analysis, or any other analysis that is capable of identifying one or more periodic components in a signal.

Periodicity strength is weakly but significantly correlated with y-fraction

In some embodiments, a relevant transcription factor is one that differentially binds to DNA molecules having differing origins. As an example, it may be desirable to identify the fetal DNA fraction, which is the percentage of fetal DNA in a sample. A blood sample from a pregnant woman may include DNA molecules of maternal origin and DNA molecules of fetal origin. A transcription factor that differentially binds to DNA molecules of maternal versus fetal origin may then be used to determine an origin of a sample. One of ordinary skill will understand that the present disclosure is not limited to differentiating between maternal and fetal tissue, and is also applicable to differentiating between other types of tissue, such as tumor versus non-tumor, diseased vs. non-diseased, host vs. non-host (for organ transplants or other exogenous sources) and lymphocyte vs. non-lymphocyte tissue.

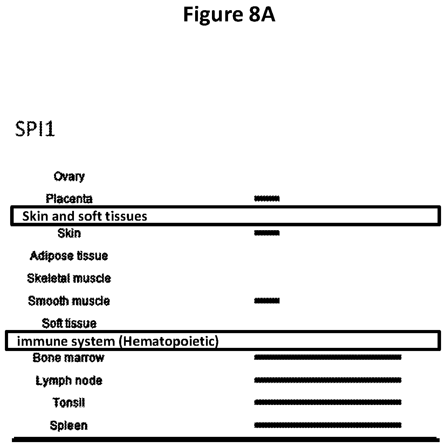

In one example, a transcription factor may preferentially bind to DNA molecules of maternal origin, and may not preferentially bind to DNA molecules of fetal origin. Placental tissue may be used as a proxy for fetal tissue, while tissue from the immune system may be used as a proxy for maternal tissue. Typically, 2-20% of circulating cell-free DNA in a blood sample from a pregnant woman is from the placenta. The length of the bars in the chart in Figure 8A is indicative of a relative preference for SPI1 to bind to various types of tissue, and indicates that SPI1 preferentially binds to DNA molecules of maternal origin, as compared to those of fetal origin.

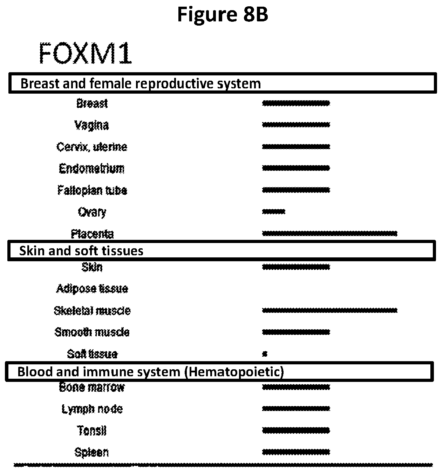

The same analysis may be performed for various transcription factors. Figure 8B indicates that FOXM1 preferentially binds to DNA molecules of fetal (i.e., placenta) origin, as compared to maternal (i.e., immune system) origin.

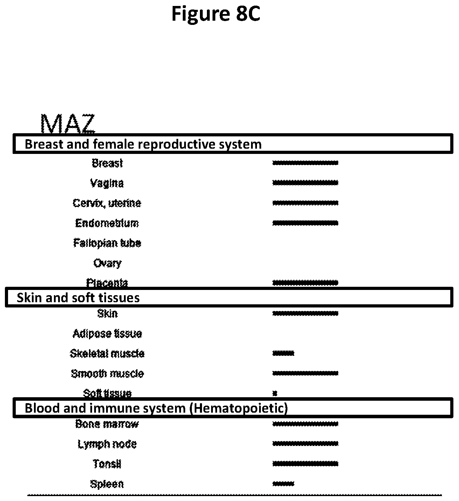

Other transcription factors may not differentially bind to DNA molecules of fetal or maternal origin. Figure 8C indicates that MAZ does not preferentially bind to either DNA molecules of fetal (i.e., placenta) origin, as compared to maternal (i.e., immune system) origin.

The observations above may be used to identify transcription factors that (1) preferentially bind to DNA molecules of maternal origin compared to those of fetal origin, such as SPI 1, (2) preferentially bind to DNA molecules of fetal origin compared to those of maternal origin, such as FOXM1, or (3) do not preferentially bind to DNA molecules of maternal or fetal origin, such as MAZ.

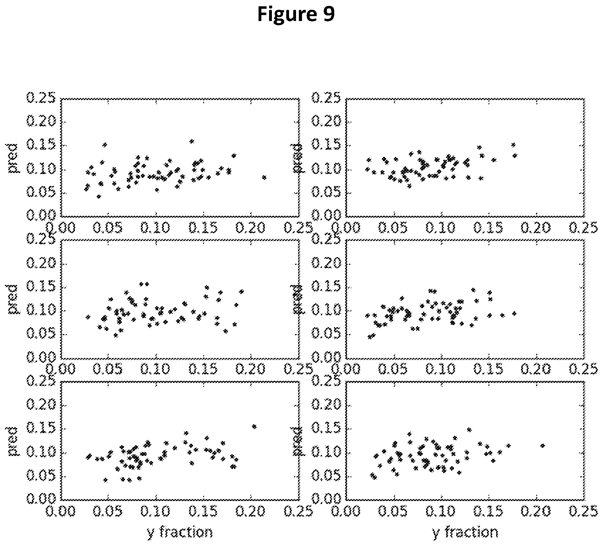

As was described above, the strength of periodicity in the coverage map is indicative of a strength of transcription factor binding. Blood samples are taken from women who were pregnant with male fetuses. In this case, the Y-fraction may be used as a proxy for fetal fraction, and the strength of periodicity in the coverage map is compared to the measured Y-fraction from the samples. The below regressions indicate a weak but highly significant (p-values were 7E-7, 5E-6, 1E-5) correlation between the strength of periodicity and the y-fraction. Transcription factors having significant correlations to Y-fraction include at least SPI1, FOXM1, MAZ, CTCFL, ARID3A, CTCF, and CNF143. As seen in Figure 9, the x axis represents the fetal fraction as predicted by the amount of Y chromosomal material in the plasma fraction. The Y axis represents the predicted fraction. Each panel represents one train/test split in a cross validation analysis, wherein the data is divided into six parts. The first part is used to evaluate a model that is trained on the other five parts, then the second part is used to evaluate a model that is trained on parts 1 and 3-6, and so on, until each of the six parts has been used to evaluate a model trained on the remaining data. Each panel shows the performance of the model on a different set of testing data. There are six panels because there are six splits, and hence six test sets. Similarly, fetal fraction can be calculated by accumulating training data, with examples of samples with known constituent fetal fractions, and fitting a model to the data. This model then can be used to predict fractions for new samples. Exemplary models include, but are not limited to, regression models. Exemplary regression models include, but are not limited to, multivariate regressions, such as least squares regressions. These data show that, by finding a correlation between a marker and a DNA origin, DNA organization can be used to determine the origin of the DNA. In certain embodiments, databases can be used to find protein binding sites useful in the embodiments of the invention.

The systems and methods of the present disclosure have several advantages over existing methods of determining fetal fraction. First, the present disclosure describes a way to determine fetal fraction on the basis of single end sequencing data, which is cheaper and faster than paired end data. Second, the signal-to-noise ratio is improved when many binding sites are averaged for each transcription factor, and the spectral analysis described herein that measures the strength of nucleosome positioning allows for data with relatively low coverage to still be successfully analyzed. Third, the present disclosure offers tenability. For each transcription factor, there may be many binding sites (up to 100,000) in the genome. The particular set of binding sites that are used may be optimized for high performance in the specific discrimination or prediction task.

Che following numbered paragraphs (paras.) contain further statements of various aspects of the present invention:

1. A method for determining the presence of DNA molecules from an origin of interest in a population of DNA molecules present in a cell-free bodily fluid sample from a subject, said method comprising:

   a) obtaining a DNA sample isolated from a cell-free bodily fluid sample from the subject;
   b) determining a plurality of protein binding site sequences and their 5' and 3' flanking region sequences for each of one or more proteins, wherein at least one of the one or more proteins differentially binds to DNA molecules of differing origin;
   c) aligning at least a plurality of the determined protein binding site sequences for each of the one or more proteins;
   d) counting the number of sequencing reads starting at each nucleotide position within each 5' and 3' flanking region sequence of the aligned protein binding site sequences;
   e) generating a coverage map based on the number of counts of step d);
   f) filtering the coverage map to identify at least one periodic component within the coverage map;
   g) obtaining a metric that is representative of a strength of the at least one periodic component within the coverage map;

   wherein the computed metric is indicative of the presence of DNA molecules from the origin of interest.

2. The method of para. 1, wherein the bodily fluid sample is a blood sample.

3. The method of para. 2, wherein the blood sample is from a pregnant woman.

4. The method of any one of paras. 1-3, wherein the DNA molecules of differing origin are DNA molecules of maternal origin and DNA molecules of fetal origin.

5. The method of para. 4, wherein the computed metric is indicative of fetal DNA fraction.

6. The method of para. 1, wherein the DNA molecules of differing origin are DNA molecules of diseased cells and DNA molecules of non-diseased cells.

7. The method of para. 1, wherein the DNA molecules of differing origin are DNA molecules of a first tissue origin and DNA molecules of a second tissue origin.

8. The method of para. 1, wherein the DNA molecules of differing origin are DNA molecules of a first tissue origin and DNA molecules of leukocyte origin.

9. The method of any one of paras. 1-8, wherein the determining is performed by sequencing.

10. The method of para. 9, wherein the sequencing is massively parallel sequencing.

11. The method of para. 9, wherein the sequencing is targeted sequencing.

12. The method of any one of paras. 1-11, wherein the proteins are transcription factors and the protein binding site sequences are transcription factor binding site sequences.

13. The method of any one of paras. 1-11, wherein the proteins are nucleases and the protein binding site sequences are nuclease binding sequences.

14. The method of any one of paras. 1-13, wherein the aligning is an alignment against a genomic reference sequence.

15. The method of any one of paras. 1-14, wherein the plurality of protein binding site sequences comprises at least 500, at least 1,000, at least 1,500, at least 2,000, at least 3,000, at least 4,000, at least 5,000, at least 10,000, at least 20,000, at least 30,000, at least 40,000, at least 50,000, at least 60,000, at least 70,000, at least 80,000, at least 90,000, at least 100,000, at least 110,000, at least 120,000, at least 130,000, at least 140,000, at least 150,000, at least 160,000, at least 170,000, at least 180,000, at least 190,000, at least 200,000, at least 210,000, at least 220,000, at least 230,000, at least 240,000, at least 250,000, at least 260,000, at least 270,000, at least 280,000, at least 290,000, at least 300,000, at least 310,000, at least 320,000, at least 330,000, at least 340,000, at least 350,000, at least 360,000, at least 370,000, at least 380,000, at least 390,000, at least 400,000, at least 410,000, at least 420,000, at least 430,000, at least 440,000, at least 450,000, at least 460,000, at least 470,000, at least 480,000, at least 490,000, or at least 500,000 protein binding site sequences.

16. The method of any one of paras. 1-15, wherein the one or more proteins is two proteins.

17. The method of any one of paras. 1-15, wherein the one or more proteins is three proteins.

18. The method of any one of paras. 1-15, wherein the one or more proteins is four proteins.

19. The method of any one of paras. 1-15, wherein the one or more proteins is five proteins.

20. The method of any one of paras. 1-15, wherein the one or more proteins is 6, 7, 8, 9, 10, 11, 12, 13, 14, 15, 16, 17, 18, 19, 20, or 21 or more proteins.

21. The method of any one of paras. 1-20, wherein the 5' and 3' flanking region sequences are at least 200, at least 300, at least 400, at least 500, at least 600, at least 700, at least 800, at least 900, at least 1,000, at least 1,100, at least 1,200, at least 1,300, at least 1,400, at least 1,500, or at least 2,000 base pairs.

22. The method of any one of paras. 1-21, wherein the filtering of step f) comprises computing a spectral frequency transform of the coverage map and identifying a power of the spectral frequency transform within a frequency band.

23. The method of para. 22, wherein the frequency band includes frequencies corresponding to spacings of 130 to 250 base pairs.

24. The method of any one of paras. 22 or 23, wherein the metric is a ratio between the power of the spectral frequency transform within a frequency band and an overall power of the spectral frequency transform.

25. The method of para. 24, wherein the power of the spectral frequency transform is computed by integrating the spectral frequency transform within the frequency band, and the overall power of the spectral frequency transform is computed by integrating the spectral frequency transform over all frequencies.

26. The method of any one of paras. 1-25, wherein the at least one periodic component is indicative of aligned positions across nucleosomes, such that a local maximum in the at least one periodic component is indicative of an absence of nucleosomes at the corresponding nucleotide position, and a local minimum in the at least one periodic component is indicative of a presence of nucleosomes at the corresponding nucleotide position.

27. The method of any one of paras. 1-26, wherein the metric is a signal-to-noise ratio that is computed from the filtered coverage map.

28. The method of any one of paras. 1-27, further comprising determining a proportion of DNA molecules from two or more origins of interest.

29. The method of para. 28, wherein the two or more origins of interest are tissues.

**Claims**

1. A method for determining the presence of DNA molecules from an origin of interest in a population of DNA molecules present in a cell-free bodily fluid sample from a subject, said method comprising:

    a) obtaining a DNA sample isolated from a cell-free bodily fluid sample from the subject;
    b) determining, by sequencing, a plurality of protein binding site sequences and their 5' and 3' flanking region sequences for each of one or more proteins, wherein at least one of the one or more proteins differentially binds to DNA molecules of the origin of interest and DNA molecules of differing origins;
    c) aligning at least 500 of the determined protein binding site sequences for each of the one or more proteins;
    d) counting the number of sequencing reads starting at each nucleotide position within each 5' and 3' flanking region sequence of the aligned protein binding site sequences, wherein the 5' and 3' flanking region sequences are at least 500 base pairs;
    e) generating a coverage map based on the number of counts of step d);
    f) filtering the coverage map to identify at least one periodic component within the coverage map, wherein the filtering comprises computing a spectral frequency transform of the coverage map and identifying a power of the spectral frequency transform within a frequency band, wherein the frequency band includes frequencies corresponding to spacings of 130 to 250 base pairs;
    g) obtaining a computed metric, wherein said computed metric is percentage spectral power computed as a ratio between the power of the spectral frequency transform within the frequency band computed by integrating the spectral frequency transform within the frequency band and an Overall power of the spectral frequency transform computed by integrating the spectral frequency transform over all frequencies F according to the expression:

$$\frac{\int_a^b |F(\omega)|d\omega}{\int |F(\omega)|d\omega}$$

    where F(w) corresponds to the Fourier coefficient for frequency w, a corresponds to a first edge of the frequency band, and b corresponds to a second edge of the frequency band;
    wherein a computed metric that is statistically different from a predetermined set of values is indicative of the presence of DNA molecules from the origin of interest.

2. The method of claim 1 , wherein the bodily fluid sample is a blood sample.

3. The method of claim 2, wherein the blood sample is from a pregnant woman.

4. The method of any one of claims 1-3, wherein the DNA molecules of differing origin are DNA molecules of maternal origin and DNA molecules of fetal origin, and wherein the computed metric is indicative of fetal DNA fraction.

5. The method of claim 1 , wherein the DNA molecules of differing origin are DNA molecules of diseased cells and DNA molecules of non-diseased cells.

6. The method of claim 1 , wherein the DNA molecules of differing origin are DNA molecules of a first tissue origin and DNA molecules of a second tissue origin or DNA molecules of leukocyte origin.

7. The method of any preceding claim, wherein the sequencing is targeted sequencing.

8. The method of any one of claims 1-7, wherein said proteins are nucleases and the protein binding site sequences are nuclease binding sequences.

9. The method of any one of claims 1-8, wherein the plurality of protein binding site sequences comprises at least 1,000, at least 1,500, at least 2,000, at least 3,000, at least 4,000, at least 5,000, at least 10,000, at least 20,000, at least 30,000, at least 40,000, at least 50,000, at least 60,000, at least 70,000, at least 80,000, at least 90,000, at least 100,000, at least 110,000, at least 120,000, at least 130,000, at least 140,000, at least 150,000, at least 160,000, at least 170,000, at least 180,000, at least 190,000, at least 200,000, at least 210,000, at least 220,000, at least 230,000, at least 240,000, at least 250,000, at least 260,000, at least 270,000, at least 280,000, at least 290,000, at least 300,000, at least 310,000, at least 320,000, at least 330,000, at least 340,000, at least 350,000, at least 360,000, at least 370,000, at least 380,000, at least 390,000, at least 400,000, at least 410,000, at least 420,000, at least 430,000, at least 440,000, at least 450,000, at least 460,000, at least 470,000, at least 480,000, at least 490,000, or at least 500,000 protein binding site sequences.

10. The method of any one of claims 1-9, wherein the 5' and 3' flanking region sequences are least 600, at least 700, at least 800, at least 900, at least 1,000, at least 1,100, at least 1,200, at least 1,300, at least 1,400, at least 1,500, or at least 2,000 base pairs.

11. The method of any one of claims 1-10, wherein the at least one periodic component is indicative of aligned positions across nucleosomes, such that a local maximum in the at least one periodic component is indicative of an absence of nucleosomes at the corresponding nucleotide position, and a local minimum in the at least one periodic component is indicative of a presence of nucleosomes at the corresponding nucleotide position.

12. The method of any one of claims 1-11, further comprising determining a proportion of DNA molecules from two or more origins of interest.

Figure 1

**Figure 2A**

exposed for cutting

protected from cutting

Nucleo-some

Nucleo-some

TF

**Figure 2B**

**Figure 3**

**Figure 4**

Figure 5

**A**

CTCF

**B**

E2F1

**C**

GTF2F1

**D**

EBF1

# Figure 6

**Figure 6**

**Figure 7A**

Figure 7B

# Figure 8A

SPI1

Figure 8B

# FOXM1

| Breast and female reproductive system | |
|---|---|
| Breast | ▬▬▬▬▬▬ |
| Vagina | ▬▬▬▬▬▬ |
| Cervix, uterine | ▬▬▬▬▬ |
| Endometrium | ▬▬▬▬▬ |
| Fallopian tube | ▬▬▬▬▬ |
| Ovary | ▬▬ |
| Placenta | ▬▬▬▬▬▬▬▬▬▬ |

| Skin and soft tissues | |
|---|---|
| Skin | ▬▬▬▬▬▬ |
| Adipose tissue | |
| Skeletal muscle | ▬▬▬▬▬▬▬▬▬▬ |
| Smooth muscle | ▬▬▬▬▬ |
| Soft tissue | ▪ |

| Blood and immune system (Hematopoietic) | |
|---|---|
| Bone marrow | ▬▬▬▬▬ |
| Lymph node | ▬▬▬▬▬ |
| Tonsil | ▬▬▬▬▬ |
| Spleen | ▬▬▬▬▬ |

EP 4 180 532 A1

Figure 8C

## MAZ

| Breast and female reproductive system | |
|---|---|
| Breast | ▬▬▬▬ |
| Vagina | ▬▬▬▬ |
| Cervix, uterine | ▬▬▬▬ |
| Endometrium | ▬▬▬ |
| Fallopian tube | |
| Ovary | |
| Placenta | ▬▬▬▬▬ |

| Skin and soft tissues | |
|---|---|
| Skin | ▬▬▬▬ |
| Adipose tissue | |
| Skeletal muscle | ▬▬ |
| Smooth muscle | ▬▬▬ |
| Soft tissue | ▪ |

| Blood and immune system (Hematopoietic) | |
|---|---|
| Bone marrow | ▬▬▬▬ |
| Lymph node | ▬▬▬▬ |
| Tonsil | ▬▬▬▬ |
| Spleen | ▬▬ |

EP 4 180 532 A1

Figure 9

Europäisches
Patentamt

European
Patent Office

Office européen
des brevets

# EUROPEAN SEARCH REPORT

**Application Number**

**EP 22 20 3021**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2011/090556 A1 (VERINATA HEALTH INC [US]; RAVA RICHARD P [US]; CHUU YUE-JEN [US]; CHIN) 28 July 2011 (2011-07-28) * [0002], [0009],[00117],[0156] and fig. 2 [0072] * | 1-12 | INV. C12Q1/68 C12Q1/6869 |
| A | WO 2014/189957 A2 (UNIV LELAND STANFORD JUNIOR [US]) 27 November 2014 (2014-11-27) * p. 2 1. 2 to p. 3 1. 32 and claims 1-11 and claims 16-18 * | 1-12 | |
| A | WO 2014/043763 A1 (UNIV HONG KONG CHINESE [CN]; CHIU ROSSA WAI KWUN [CN]) 27 March 2014 (2014-03-27) * [0003], [008] and [009] * | 1-12 | |
| A | VLADIMIR B. TEIF ET AL: "Nucleosome repositioning links DNA (de)methylation and differential CTCF binding during stem cell development", GENOME RESEARCH, vol. 24, no. 8, 8 May 2014 (2014-05-08), pages 1285-1295, XP055338846, US ISSN: 1088-9051, DOI: 10.1101/gr.164418.113 * abstract, Fig. 1 and Fig. 5 * | 1-12 | **TECHNICAL FIELDS SEARCHED (IPC)** <br><br> C12Q |

The present search report has been drawn up for all claims

| Place of search | Date of completion of the search | Examiner |
|---|---|---|
| Munich | 29 March 2023 | Lapopin, Laurence |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**EP 4 180 532 A1**

## ANNEX TO THE EUROPEAN SEARCH REPORT
## ON EUROPEAN PATENT APPLICATION NO.

EP 22 20 3021

This annex lists the patent family members relating to the patent documents cited in the above-mentioned European search report.
The members are as contained in the European Patent Office EDP file on
The European Patent Office is in no way liable for these particulars which are merely given for the purpose of information.

29-03-2023

| Patent document cited in search report | | Publication date | Patent family member(s) | | Publication date |
|---|---|---|---|---|---|
| WO 2011090556 | A1 | 28-07-2011 | US | 2012010085 A1 | 12-01-2012 |
| | | | US | 2012214678 A1 | 23-08-2012 |
| | | | US | 2017037474 A1 | 09-02-2017 |
| | | | US | 2020385810 A1 | 10-12-2020 |
| | | | WO | 2011090556 A1 | 28-07-2011 |
| WO 2014189957 | A2 | 27-11-2014 | AU | 2014268710 A1 | 03-12-2015 |
| | | | AU | 2019200289 A1 | 07-02-2019 |
| | | | AU | 2021229232 A1 | 07-10-2021 |
| | | | CA | 2909972 A1 | 27-11-2014 |
| | | | CN | 105339503 A | 17-02-2016 |
| | | | CN | 111394426 A | 10-07-2020 |
| | | | EP | 2999792 A2 | 30-03-2016 |
| | | | EP | 3470530 A1 | 17-04-2019 |
| | | | EP | 3828285 A1 | 02-06-2021 |
| | | | HK | 1222416 A1 | 30-06-2017 |
| | | | JP | 6618894 B2 | 11-12-2019 |
| | | | JP | 6977014 B2 | 08-12-2021 |
| | | | JP | 2016518860 A | 30-06-2016 |
| | | | JP | 2020039346 A | 19-03-2020 |
| | | | JP | 2022024028 A | 08-02-2022 |
| | | | SG | 11201508985V A | 30-12-2015 |
| | | | US | 10150995 B1 | 11-12-2018 |
| | | | US | 11519032 B1 | 06-12-2022 |
| | | | US | 2016060691 A1 | 03-03-2016 |
| | | | US | 2018327838 A1 | 15-11-2018 |
| | | | US | 2019032130 A1 | 31-01-2019 |
| | | | US | 2019040464 A1 | 07-02-2019 |
| | | | US | 2019309362 A1 | 10-10-2019 |
| | | | US | 2019316196 A1 | 17-10-2019 |
| | | | US | 2020332359 A1 | 22-10-2020 |
| | | | US | 2021285045 A1 | 16-09-2021 |
| | | | US | 2022325342 A1 | 13-10-2022 |
| | | | WO | 2014189957 A2 | 27-11-2014 |
| WO 2014043763 | A1 | 27-03-2014 | AU | 2013317708 A1 | 09-04-2015 |
| | | | AU | 2017251832 A1 | 16-11-2017 |
| | | | AU | 2020200128 A1 | 30-01-2020 |
| | | | AU | 2022201026 A1 | 10-03-2022 |
| | | | BR | 112015006183 A2 | 04-07-2017 |
| | | | BR | 122021021825 B1 | 24-05-2022 |
| | | | CA | 2884066 A1 | 27-03-2014 |
| | | | CA | 3122779 A1 | 27-03-2014 |
| | | | CN | 104781422 A | 15-07-2015 |
| | | | DK | 2898100 T3 | 26-02-2018 |
| | | | DK | 3354747 T3 | 19-04-2021 |

EPO FORM P0459

For more details about this annex : see Official Journal of the European Patent Office, No. 12/82

**ANNEX TO THE EUROPEAN SEARCH REPORT
ON EUROPEAN PATENT APPLICATION NO.**

EP 22 20 3021

29-03-2023

| Patent document cited in search report | Publication date | Patent family member(s) | Publication date |
|---|---|---|---|
| | | EA 201500327 A1 | 30-07-2015 |
| | | EP 2898100 A1 | 29-07-2015 |
| | | EP 3354747 A1 | 01-08-2018 |
| | | EP 3536807 A1 | 11-09-2019 |
| | | EP 3839065 A1 | 23-06-2021 |
| | | EP 4056712 A1 | 14-09-2022 |
| | | ES 2665273 T3 | 25-04-2018 |
| | | ES 2862726 T3 | 07-10-2021 |
| | | HK 1206797 A1 | 15-01-2016 |
| | | HK 1207124 A1 | 22-01-2016 |
| | | HK 1257868 A1 | 01-11-2019 |
| | | HR P20210716 T1 | 11-06-2021 |
| | | HU E053822 T2 | 28-07-2021 |
| | | IL 268512 A | 26-09-2019 |
| | | JP 6317354 B2 | 25-04-2018 |
| | | JP 6683752 B2 | 22-04-2020 |
| | | JP 6985753 B2 | 22-12-2021 |
| | | JP 2015536639 A | 24-12-2015 |
| | | JP 2018121644 A | 09-08-2018 |
| | | JP 2020108400 A | 16-07-2020 |
| | | JP 2022031753 A | 22-02-2022 |
| | | KR 20150082228 A | 15-07-2015 |
| | | KR 20200085928 A | 15-07-2020 |
| | | KR 20210120124 A | 06-10-2021 |
| | | KR 20220065034 A | 19-05-2022 |
| | | LT 3354747 T | 12-04-2021 |
| | | MX 360034 B | 19-10-2018 |
| | | NZ 706269 A | 26-08-2016 |
| | | NZ 717423 A | 25-08-2017 |
| | | PH 12015500547 A1 | 04-05-2015 |
| | | PL 2898100 T3 | 29-06-2018 |
| | | PL 3354747 T3 | 26-07-2021 |
| | | PT 2898100 T | 26-02-2018 |
| | | PT 3354747 T | 07-05-2021 |
| | | SG 10201705198U A | 28-07-2017 |
| | | SG 11201501927V A | 29-04-2015 |
| | | TW 201418474 A | 16-05-2014 |
| | | TW 201907013 A | 16-02-2019 |
| | | TW 202043483 A | 01-12-2020 |
| | | WO 2014043763 A1 | 27-03-2014 |
| | | ZA 201501772 B | 26-10-2016 |

EPO FORM P0459

For more details about this annex : see Official Journal of the European Patent Office, No. 12/82

**EP 4 180 532 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62252965